# EUROPEAN PATENT APPLICATION

(11) **EP 4 349 977 A1**
(43) Date of publication of application: **10.04.2024**
(21) Application number: 21942622.8
(22) Date of filing: 08.09.2021
(51) Int. Cl.: C12N 9/12, C12Q 1/68

(54) **THERMOSTABLE B-FAMILY DNA POLYMERASE MUTANT AND APPLICATION THEREOF**

(30) Priority: 26.05.2021 WO PCT/CN2021/096002
(71) Applicant: BGI Shenzhen, Shenzhen, Guangdong 518083 (CN)
(72) Inventor: ZHAI, Lili, Shenzhen, Guangdong 518083 (CN); XIE, Qingqing, Shenzhen, Guangdong 518083 (CN); CAO, Ruyin, Shenzhen, Guangdong 518083 (CN); ZHENG, Yue, Shenzhen, Guangdong 518083 (CN); DONG, Yuliang, Shenzhen, Guangdong 518083 (CN); ZHANG, Wenwei, Shenzhen, Guangdong 518083 (CN); XU, Xun, Shenzhen, Guangdong 518083 (CN); CHEN, Zhuo, Shenzhen, Guangdong 518083 (CN)
(74) Representative: Schwarz & Partner Patentanwälte GmbH
(86) International application number: PCT/CN2021/117113
(87) International publication number: WO 2022/247055

(57) **Abstract**

Provided are a thermostable B-family DNA polymerase mutant and an application thereof. The thermostable B-family DNA polymerase mutant is provided to mutate amino acid residues in at least two of three sites of group A, i.e., 408, 409, and 410 in an amino acid sequence of thermostable B-family DNA polymerases to obtain proteins having DNA polymerase activity. A-group sites (408/409/410), B-group sites (451/485), C-group sites (389/383/384), D-group sites (589/67/680), E-group sites (491/493/494/497), and F-group sites (474/478/486/480/484) of several B-family DNA polymerases are analyzed to design the mutant. Compared with non-mutant DNA polymerases, several groups of mutant DNA polymerases exhibit a strong polymerization capability in catalyzing 3' blocking-modified nucleotides and provide a basis for the sustainable development of high-throughput sequencing.

## Description

### FIELD

The present disclosure relates to the technical field of enzyme engineering, and in particular to a thermostable B-family DNA polymerase mutant and use thereof.

### BACKGROUND

DNA polymerase is responsible for quick and accurate DNA replication, which is important for enabling organisms to transmit genetic information accurately and maintain the genetic material stability. Similar to the human hand, the general fold-framework of DNA polymerase has three domains, the palm, thumb and finger. Although structures of the finger and the palm domains may vary greatly from different kinds of DNA polymerases, the palm domains for catalysis among which are relatively similar. There is a great demand in incorporating nucleotide analogs or modified nucleotides, especially specific dNTPs with 3'-end blocking modifications.

The thermostable B-family DNA polymerases include KOD DNA polymerases (*Thermococcus kodakaraensis*)*,* 9°N DNA polymerases (Thermophilic species), TGO DNA polymerases (*Thermococcus gorgonarius*), TOK DNA polymerases (*Desulfurococcus sp.* Tok), Vent DNA polymerases (*Thermococcus litoralis*), JDF-3, pfu DNA polymerases (*Pyrococcusfuriosis*) and so on.

There is a wide range of application for the thermostable B-family DNA polymerases able to incorporate specific dNTPs with blocking modifications at the 3' end into DNA templates. One of the most important applications is genome sequencing, e.g., high-throughput sequencing. The nucleotides with modifications at the hydroxyl group at 3' end of the pentose are used to block the incorporation of other nucleotides in the high-throughput sequencing, and thus the nucleotides with the blocking groups at the 3' end enable the incorporation of nucleotides into a polynucleotide strand in a controlled manner. Specifically, after each nucleotide with the blocking group at the 3' end is incorporated, the presence of such a nucleotide prevents the incorporation of other nucleotides into the strand. Further, with the removal of the blocking group, the hydroxyl group at 3' end is restored to a natural free state for incorporation of the next nucleotide.

Therefore, it has become a research highlight to search modified thermostable B-family DNA polymerases suitable for incorporating specific dNTPs with 3' end blocking modifications into DNA templates.

### SUMMARY

An object of the present disclosure is to provide a thermostable B-family DNA polymerase mutant protein.

The provided mutant protein based on a thermostable B-family DNA polymerase and having DNA polymerase activity is obtained by mutating at least two amino acid residues in an amino acid sequence of the thermostable B-family DNA polymerase at positions selected from the following three positions in group A: positions 408, 409, and 410.

The mutant protein having DNA polymerase activity, based on the mutant protein of group A, may be further mutated at least one amino acid residue at a position(s) selected from positions 451 and 485 in group B in the amino acid sequence of the thermostable B-family DNA polymerase.

The mutant protein having DNA polymerase activity, based on the mutant of groups A and B, may be further mutated at least one amino acid residue at a position(s) selected from three positions 383, 384, and 389 in group C in the amino acid sequence of the thermostable B-family DNA polymerase.

The mutant protein having DNA polymerase activity, based on the mutant of groups A and B, or groups A, B and C, may be further mutated at least one amino acid residue at a position(s) selected from three positions 589, 676, and 680 in group D in the amino acid sequence of the thermostable B-family DNA polymerase.

The mutant protein having DNA polymerase activity, based on the mutant of groups A and B, may be further mutated at least one amino acid residue at a position(s) selected from four positions 491, 493, 494, and 497 in group E in the amino acid sequence of the thermostable B-family DNA polymerase.

The mutant protein having DNA polymerase activity, based on the mutant of groups A and B, may be further mutated at least one amino acid residue at a position(s) selected from five positions 474, 478, 480, 484 and 486 in the F group in the amino acid sequence of the thermostable B-family DNA polymerase.

The mutant protein having DNA polymerase activity, based on the mutant of groups A and B, may be further mutated an amino acid residue at position 93 in the amino acid sequence of the thermostable B-family DNA polymerase.

Positions as described above correspond to amino acid sites in the amino acid sequence of the thermostable B-family DNA polymerase respectively, for example, position 408 is the 408th amino acid site of the amino acid sequence of the thermostable B-family DNA polymerase.

In embodiments of the present disclosure, the following thermostable B-family DNA polymerase is taken as an example: a KOD DNA polymerase, 9°N DNA polymerase, TGO DNA polymerase, TOK DNA polymerase, or a mutant thereof.

The KOD DNA polymerase is of an amino acid sequence set forth in SEQ ID NO: 1 in the sequence listing; and a mutant of the KOD DNA polymerase is of an amino acid sequence set forth in SEQ ID NO: 5 in the sequence listing. The 9°N DNA polymerase is of an amino acid sequence set forth in SEQ ID NO: 2 in the sequence listing, the TGO DNA polymerase is of an amino acid sequence set forth in SEQ ID NO: 3 in the sequence listing, and the TOK DNA polymerase is of an amino acid sequence set forth in SEQ ID NO: 4 in the sequence listing.

The present disclosure relates to mutant proteins of four thermostable B-family DNA polymerases, which are four proteins.

In an embodiment, the DNA polymerase is a mutant protein obtained with a single-site mutation or multi-site combinatorial mutation based on a thermostable B-family DNA polymerase, the KOD DNA polymerase (*Thermococcus kodakaraensis*)*,* which is of an amino acid sequence set forth in SEQ ID NO: 1.

In an embodiment, the DNA polymerase is a mutant protein obtained with a single-site mutation or multi-site combinatorial mutation based on a thermostable B-family DNA polymerase, the 9°N DNA polymerase (*Thermophilic species*), which is of an amino acid sequence set forth in SEQ ID NO: 2.

In an embodiment, the DNA polymerase is a mutant protein obtained with a single-site mutation or multi-site combinatorial mutation based on a thermostable B-family DNA polymerase, the TGO DNA polymerase (*Thermococcus gorgonarius*), which is of an amino acid sequence set forth in SEQ ID NO: 3.

In an embodiment, the DNA polymerase is a mutant protein obtained with a single-site mutation or multi-site combinatorial mutation based on a thermostable B-family DNA polymerase, the TOK DNA polymerase (*Desulfurococcus sp. Tok*), which is of an amino acid sequence set forth in SEQ ID NO: 4.

In embodiments of the present disclosure, the mutant protein may be in any one form with the following combinatorial mutations:
1. a mutant protein having DNA polymerase activity obtained by mutating the amino acid residues at positions including or corresponding to the following two positions in the amino acid sequence of the thermostable B-family DNA polymerase, where said two positions are positions 408, 409 in group A of the amino acid sequence of the thermostable B-family DNA polymerase;
2. a mutant protein having DNA polymerase activity obtained by mutating the amino acid residues at positions including or corresponding to the following three positions in the amino acid sequence of the thermostable B-family DNA polymerase, where said three positions are any one of the following position combinations: positions 408 and 409 in group A and position 485 in group B; positions 408, 409 and 410 in group A; and positions 408 and 409 group A and position 491 in group E, of the amino acid sequence of the thermostable B-family DNA polymerase;
3. a mutant protein having DNA polymerase activity obtained by mutating the amino acid residues at positions including or corresponding to the following four positions in the amino acid sequence of the thermostable B-family DNA polymerase, where said four positions are any one of the following position combinations: positions 408 and 409 in group A, and positions 451 and 485 in group B; positions 408 and 409 in group A, position 485 in group B, and position 497 in group E; positions 408, 409 and 410 in group A, and position 485 in group B; and positions 408 and 409 in group A, position 451 in group B, and position 93, of the amino acid sequence of the thermostable B-family DNA polymerase;
4. a mutant protein having DNA polymerase activity obtained by mutating the amino acid residues at positions including or corresponding to the following five positions in the amino acid sequence of the thermostable B-family DNA polymerase, where said five positions are any one of the following position combinations: positions 408, 409, 410 in group A, and positions 451, 485 in group B; positions 408, 409 in group A, positions 451, 485 in group B and position 494 in group E; positions 408, 409 in group A, and positions 451, 485, 486 in group B; positions 408, 409 in group A, positions 451, 485 in group B, and position 486 in group F; or positions 408, 409 in group A, positions 451, 485 in group B and position 480 in group F; positions 408, 409 in group A, positions 451, 485 in group B, and position 484 in group F; and positions 408, 409 in group A, positions 451, 485 in group B, and position 478 in group F, of the amino acid sequence of the thermostable B-family DNA polymerase;
5. a mutant protein having DNA polymerase activity obtained by mutating the amino acid residues at positions including or corresponding to the following six positions in the amino acid sequence of the thermostable B-family DNA polymerase, where said six positions are any one of the following position combinations: positions 408, 409 in group A, position 485 in group B, and positions 589, 676, 680 in group D; positions 408, 409 in group A, position 485 in group B, and positions 389, 383, 384 in group C; positions 408, 409 in group A, position 485 in group B, and positions 491, 494, 497 in group E; positions 408, 409 in group A, positions 451, 485 in group B, and positions 493, 497 in group E; and positions 408, 409 in group A, positions 451, 485 in group B, and positions 474, 478 in group F, of the amino acid sequence of the thermostable B-family DNA polymerase;
6. a mutant protein having DNA polymerase activity is obtained by mutating the amino acid residues at positions including or corresponding to the following seven positions in the amino acid sequence of the thermostable B-family DNA polymerase, where said seven positions are any one of the following position combinations: positions 408, 409 in group A, positions 485, 451 in group B, and positions 589, 676 and 680 in group D; positions 408, 409 in group A, positions 485, 451 in group B, and positions 389, 383 and 384 in group C; and positions 408, 409 in group A, positions 485, 451 in group B, and positions 493, 497 and 491 in group E, of the amino acid sequence of the thermostable B-family DNA polymerase;
7. a mutant protein having DNA polymerase activity obtained by mutating the amino acid residues at positions including or corresponding to the following eight positions in the amino acid sequence of the thermostable B-family DNA polymerase, where said eight positions are any one of the following position combination: positions 408, 409 in group A, positions 485, 451 in Group B, positions 493, 497, 491 and 497 in group E, of the amino acid sequence of the thermostable B-family DNA polymerase;
8. a mutant protein having DNA polymerase activity is obtained by mutating the amino acid residues at positions including or corresponding to the following nine positions in the amino acid sequence of the thermostable B-family DNA polymerase, where said nine positions are any one of the following position combination: positions 408, 409 in group A, position 485 in group B, position s 589, 676, 680 in group D, and positions 389, 383 and 384 in group C of the amino acid sequence of the thermostable B-family DNA polymerase; and
9. a mutant protein having DNA polymerase activity obtained by mutating the amino acid residues at positions including or corresponding to the following ten positions in the amino acid sequence of the thermostable B-family DNA polymerase, where said ten positions are any one of the following position combination: positions 408, 409 in group A, positions 451, 485 in group B, positions 589, 676, 680 in group D, and positions 389, 383 and 384 in group C of the amino acid sequence of the thermostable B-family DNA polymerase.

According to the above mutant proteins, the amino acid residue at each of the positions is mutated as the following manners:
L at position 408 is mutated into A (alanine), I (isoleucine) or Y (tyrosine);
Y at position 409 is mutated into A (alanine);
P at position 410 is mutated into D (aspartic acid) or C (cysteine) or N (asparagine);
S at position 451 is mutated into T (threonine) or L (leucine);
E at position 485 is mutated into I (isoleucine) or L (leucine), or A at position 485 is mutated into L (leucine) or E (glutamic acid) or I (isoleucine);
V (valine) at position 93 is mutated into Q (glutamine);
I (isoleucine) at position 486 is mutated into R(arginine);
D at position 480 is mutated into T (threonine) or N (asparagine);
R (arginine) at position 484 is mutated into C (cysteine);
I (isoleucine) at position 474 is mutated into T (threonine) or K (lysine);
L (leucine) at position 478 is mutated into I (isoleucine) or F (phenylalanine) or R (arginine) / G (glycine) / H (histidine) / C (cysteine) / Y (tyrosine) / S (serine);
S (serine) at position 383 is mutated into T (threonine);
Y (tyrosine)at position 384 is mutated to F (phenylalanine);
V at position 389 is mutated to I (isoleucine);
V at position 589 is mutated into H (histidine);
T at position 676 is mutated into K (lysine);
V at position 680 is mutated to M (methionine);
A or P (proline) at position 491 is mutated into G (glycine);
Y at position 493 is mutated to F (phenylalanine);
Y at position 494 is mutated to F (phenylalanine);
Y at position 497 is mutated into V (valine) or F (phenylalanine) or L (leucine).

In some embodiments, the mutant protein above may be added with a tag sequence at an end of the amino acid sequence of the mutant protein, and the mutant protein has thermostable B-family DNA polymerase activity.

A DNA molecule encoding the above mutant protein also falls into the scope of protection of the present disclosure.

An expression cassette, a recombinant vector, a recombinant bacterium or a transgenic cell line including the above DNA molecule also falls into the scope of protection of the present disclosure.

Use of the above mutant protein in at least one of the following i to v also falls into the scope of protection of the present disclosure:
i. performing as a DNA polymerase;
ii. catalyzing DNA replication and/or DNA amplification;
iii. nucleic acid sequencing, where a specific dNTP with 3'-end blocking modification is introduced during the sequencing;
iv. preparing a product for catalyzing DNA replication and/or DNA amplification; and
v. preparing a product for nucleic acid sequencing, where the specific dNTP with 3'-end blocking modification is introduced during the sequencing.

Use of the above DNA molecule, expression cassette, recombinant vector, recombinant bacterium or transgenic cell line in at least one of the following i to v also falls into the scope of protection of the present disclosure:
i. preparing a DNA polymerase;
ii. catalyzing DNA replication and/or DNA amplification;
iii. nucleic acid sequencing, where a specific dNTP with 3'-end blocking modification is introduced during the sequencing;
iv. preparing a product for catalyzing DNA replication and/or DNA amplification; and
v. preparing a product for nucleic acid sequencing, where the specific dNTP with 3'-end blocking modification is introduced during the sequencing.

In embodiments of the present disclosure, several thermostable B-family DNA polymerases are performed with combined mutations at various positions in groups A to E, and new DNA polymerase mutant proteins are developed, all of which may be used to add nucleotides with the 3'-end blocking modification, and thus incorporating a newly designed nucleotides with the 3'-end blocking modification into the DNA template strands. The new DNA polymerase mutant proteins may further adapt to new sequencing systems for use in high-throughput sequencing.

A DNA molecule encoding the above mutant protein, which has the amino acid sequences of the DNA polymerase mutant, also falls into the scope of protection of the present disclosure.

An expression cassette, a recombinant vector, a recombinant bacterium or a transgenic cell line including the DNA molecule also falls into the scope of protection of the present disclosure.

Use of the above mutant protein in at least one of the following i to v also falls into the scope of protection of the present disclosure:
i. performing as a DNA polymerase;
ii. catalyzing DNA replication and/or DNA amplification;
iii. nucleic acid sequencing, where a specific dNTP with 3'-end blocking modification is introduced during the sequencing;
iv. preparing a product for catalyzing DNA replication and/or DNA amplification; and
v. preparing a product for nucleic acid sequencing, where the specific dNTP with 3'-end blocking modification is introduced during the sequencing.

Use of the above DNA molecule, or the above expression cassette, recombinant vector, recombinant bacterium or transgenic cell line in at least one of the following vi to xi also falls into the scope of protection of the present disclosure:
vi. preparing a DNA polymerase;
vii. catalyzing DNA replication and/or DNA amplification;
viii. nucleic acid sequencing;
ix. preparing a product of DNA polymerase;
x. preparing a product for catalyzing DNA replication and/or DNA amplification; and
xi. preparing a product for nucleic acid sequencing.

According to the above use, the sequencing is high-throughput sequencing; and the nucleic acid is a DNA or RNA or whole genome.

According to the above use, the product is a kit.

The present disclosure is further described below with reference to embodiments.

### BRIEF DESCRIPTION OF THE DRAWINGS

FIG. 1 shows a DNA strand labeled with a Cy3 fluorescent dye (DNA-CY3).
FIG. 2 shows a dTTP with 3'-end blocking modification and labeled with a Cy5 fluorescent dye.

### DETAILED DESCRIPTION

The experimental methods used in the following embodiments are conventional unless otherwise specified.

Embodiments of the disclosure will be described in detail below in connection with the Examples, but it will be appreciated by those skilled in the art that the following Examples are only intended to illustrate the present disclosure and should not be regarded as limiting the scope of the present disclosure. The reagents or instruments used, where no manufacturer is indicated, are conventional products available through the market.

The present disclosure presents a polymerase for improving the incorporation of nucleotide analogs, which in particular have blocking modifications at the 3' end. The inventors of the present disclosure identified that the analogs can be successfully incorporated into the DNA template strand, and this has been validated by high-throughput sequencing. In order to obtain a DNA polymerase that has quick catalysis rate or incorporates nucleotides with 3'-end blocking group modifications in high throughput sequencing, the present disclosure provides the following technical solutions.

The present disclosure relates to mutant proteins of four thermostable B-family DNA polymerases.

In example 1, the DNA polymerase is a mutant protein obtained with a single-site mutation or multi-site combinatorial mutation based on a thermostable B-family DNA polymerase, the KOD DNA polymerase (*Thermococcus kodakaraensis*)*,* which is of an amino acid sequence set forth in SEQ ID NO: 1.

In example 2, the DNA polymerase is a mutant protein obtained with a single-site mutation or multi-site combinatorial mutation based on a thermostable B-family DNA polymerase, the 9°N DNA polymerase (*Thermophilic species*), which is of an amino acid sequence set forth in SEQ ID NO: 2.

In example 3, the DNA polymerase is a mutant protein obtained with a single-site mutation or multi-site combinatorial mutation based on a thermostable B-family DNA polymerase, the TGO DNA polymerase (*Thermococcus gorgonarius*)*,* which is of an amino acid sequence set forth in SEQ ID NO: 3.

In example 4, the DNA polymerase is a mutant protein obtained with a single-site mutation or multi-site combinatorial mutation based on a thermostable B-family DNA polymerase, the TOK DNA polymerase (*Desulfurococcus sp. Tok*), which is of an amino acid sequence set forth in SEQ ID NO: 4.

According to the DNA polymerase mutant proteins of the following Examples 1, 2, 3 and 4, each of the above amino acid substitutions were made in the manner as shown in Table 1 below.

**Table 1 The DNA polymerase mutant proteins and mutation positions thereof**

| Mutant ID | Sequence | Mutant Information |
|---|---|---|
| Mut_K7 | Seq_1 | L408A+Y409A+ P410D |
| Mut_K8 | Seq_5 | L408I+Y409A+ P410D+S451T+E485I |
| Mut_K9 | Seq_5 | L408I+Y409A+ P410C+S451L+E485I |
| Mut_K4 | Seq_1 | L408Y+Y409A |
| Mut_K2 | Seq_1 | L4081+Y409A |
| Mut_K19 | Seq_1 | L408A+Y409A+ S451T+A485L |
| Mut_K1 | Seq_1 | L408A+Y409A+A485L |
| Mut_K3 | Seq_5 | L408A+Y409A+E485L |
| Mut_K5 | Seq_5 | L408A+Y409A+S451T+E485L |
| Mut_K6 | Seq_5 | L408A+Y409A+S451L+E485L |
| Mut_K10 | Seq_5 | L408A+Y409A+P410C+S451T+E485L |
| Mut_K11 | Seq_5 | L408A+Y409A+P410D+S451T+E485L |
| Mut_K12 | Seq_5 | L408A+Y409A+P410N+S451T+E485L |
| Mut_K13 | Seq_1 | L408I+Y409A+A485E+V589H+T676K+V680M |
| Mut_K14 | Seq_1 | L408I+Y409A+A485E+V389I+S383T+Y384F |
| Mut_K15 | Seq_1 | L408I+Y409A+A485E+S451T+V589H+T676K+V680M |
| Mut_K16 | Seq_1 | L408I+Y409A+A485E+S451L+V389I+S383T+Y384F |
| Mut_K17 | Seq_5 | L408A+Y409A+ S451T+E485L+Y493F+Y497V |
| Mut_K20 | Seq_5 | L408A+Y409A+ S451T+E485L+Y493F+Y497F |
| Mut_K18 | Seq_1 | L408A+Y409A+Y497L |
| Mut_K21 | Seq_5 | L408A+Y409A+S451T+E485L+I486R |
| Mut_K22 | Seq_5 | L408A+Y409A+S451T+E485L+I474T+L478I |
| Mut_K23 | Seq_5 | L408A+Y409A+S451T+E485L+I474T+L478F |
| Mut_K24 | Seq_5 | L408A+Y409A+E485L+S451T+D480T |
| Mut_K25 | Seq_5 | L408A+Y409A+E485I+S451T+D480N |
| Mut_K26 | Seq_5 | L408A+Y409A+E485I+S451T+R484C |
| Mut_K27 | Seq_5 | L408A+Y409A+E485L+S451T+L478R |
| Mut_K28 | Seq_5 | L408A+Y409A+E485L+S451T+I474K+L478F |
| Mut_K29 | Seq_5 | L408A+Y409A+E485L+S451T+I474K+L478R |
| Mut_K30 | Seq_5 | L408A+Y409A+E485L+S451T+I474K+L478I |
| Mut_K31 | Seq_5 | L408A+Y409A+E485L+S451T+I474K+L478G |
| Mut_K32 | Seq_5 | L408A+Y409A+E485L+S451T+I474T+L478H |
| Mut_K33 | Seq_5 | L408A+Y409A+E485L+S451T+I474T+L478C |
| Mut_K34 | Seq_5 | L408A+Y409A+E485L+S451T+I474T+L478Y |
| Mut_K35 | Seq_5 | L408A+Y409A+E485L+S451T+I474T+L478S |
| Mut_N1 | Seq_2 | L408A+Y409A+A485L |
| Mut_N2 | Seq_2 | L408A+Y409A+A485L+S451L |
| Mut_N3 | Seq_2 | L408A+Y409A+A485L+Y497L |
| Mut_N4 | Seq_2 | L408A+Y409A+P410N+A485L |
| Mut_N5 | Seq_2 | L408A+Y409A+P410D+A485L+S451L |
| Mut_N6 | Seq_2 | L408A+Y409A+A485L+V589H+T676K+V680M |
| Mut_N7 | Seq_2 | L408A+Y409A+A485L+V389I+S383T+Y384F |
| Mut_N8 | Seq_2 | L408I+Y409A+A485I+V589H+T676K+V680M+ V3891+S383T+Y384F |
| Mut_N9 | Seq_2 | L408I+Y409A+S451L+A485I+V589H+T676K+V680M+ V3891+S383T+Y384F |
| Mut_N10 | Seq_2 | L408A+Y409A+A485L+A491G+Y494F+Y497L |
| Mut_T1 | Seq_3 | L408A+Y409A |
| Mut_T2 | Seq_3 | L4081+Y409A |
| Mut_T3 | Seq_3 | L408I+Y409A+S451L+A485I |
| Mut_T4 | Seq_3 | L408I+Y409A+P491G |
| Mut_T5 | Seq_3 | L4081+Y409A+A485L |
| Mut_T6 | Seq_3 | L408A+Y409A+P410N |
| Mut_T7 | Seq_3 | V93Q+L408A+Y409A+A485L |
| Mut_O1 | Seq_4 | L408A+Y409A |
| Mut_O2 | Seq_4 | L4081+Y409A |
| Mut_O3 | Seq_4 | L408I+Y409A+S451L+A485I |

In the above table, the mutations and positions in the 3th column are amino acid substitutions based on the sequences in column 2 respectively.

### Example 1: Preparation of KOD DNA polymerase and mutant proteins thereof

According to Examples of the present disclosure, the KOD DNA polymerase and its mutants were constructed into expression vectors of Electra^{™} Cloning Reagents Kit (DNA 2.0), and subsequently purified by affinity purification with Ni columns by means of His tags.

### 1.1 Preparation of KOD DNA polymerase KOD_WT

The amino acid sequence of the KOD DNA polymerase KOD_WT was set forth as SEQ ID NO: 1 in the sequence listing.

### 1.1.1 Construction of expression vector pD441-WT for KOD_WT

According to the instructions of the Electra^{™} Cloning Reagents Kit (DNA2.0, EKT-02), a gene coding KOD DNA polymerase, which was fused with His tags at the N-terminal, was recombined into the vector pD441-pelB (DNA2.0, pD441-pelB) to construct the recombinant vector pD441-WT. The coding gene of KOD_WT fused with His tags was induced by IPTG through the lactose (Lac) operator in the vector, to express the KOD DNA polymerase fused with His tags.

After the recombinant vector pD441-WT was successfully constructed, plasmids were extracted and sequenced to confirm correct sequence information.

### 1.1.2 Construction of recombinant bacterium

The recombinant vector pD441-WT was introduced into *E.coli* BL21 competent cells (purchased from TransGen Biotech Co., Ltd.), and then the cells were coated on a plate containing kanamycin with 50 µg/ml for screening of positive colonies. The plasmids of the screened positive bacteria were extracted for verification, and named as BL21/pD441-WT.

### 1.1.3 Preparation of KOD_WT protein

Single colony of BL21/pD441-WT was picked and cultured in 50 ml LB liquid medium (containing 50 µg/ml of Kan) at 37 °C, 220 rpm/min overnight. On the next day, the colony was transferred to 1 L LB liquid medium (containing 50 µg/ml of Kan) at a dilution of 1:100, and incubated in a shaker at 37 °C and 220 rpm/min, until OD600 of the bacterial suspension to be 0.5-0.8. Then IPTG was added into the bacterial suspension at a final concentration of 0.5 mM and incubated overnight at 25 °C to induce the expression of the fusion protein, thereby obtaining the induced BL21/pD441-WT bacterial suspension.

The above induced BL21/pD441-WT bacterial suspension was centrifuged at 8000 rpm/min for 10 min, to collect pelleted bacteria by discarding the supernatant. The bacteria were resuspended in buffer 1 which is at pH 7.0 and contains 50 mM KPO4, 500 mM NaCl, 10 mM imidazole, 5% Glycerol, and PMSF at a final concentration of 0.5 mM was added additionally. Then the bacteria was disrupted by an ultrasonic cell disruptor set with a working time of 40 min, power of 200 W, ultrasonic disruption for 1 second and stop 2 seconds alternately, and an alarm temperature for 15 °C. After the ultrasonic disruption, the solution of disrupted bacteria was placed in a water bath at 80 °C for 30 min, and was regularly mixed during the bath for uniform heat. The heated solution of disrupted bacteria was centrifuged at 12,000 rpm/min for 30 min at 4 °C, and the supernatant was filtered through 0.22 µm filter membrane. The filtrate was collected as the cell crude extract.

The cell crude extract was loaded at a flow rate of 3 mL/min and purified by affinity chromatography on an equilibrated Ni column (affinity chromatography pre-packed column HisTrap FF, 5 ml, GE healthcare). The loaded column was equilibrated with 10 CV of the buffer 1, and subjected to a linear elution with 10 CV of buffer 2 which is at pH 7.0 and contains 50 mM KPO4, 500 mM NaCl, 500 mM imidazole and 5% GHG. The eluent of the Ni column affinity chromatography corresponding to the peak greater than or equal to 100 mAU was collected.

The eluent corresponding to the peak greater than or equal to 100 mAU was diluted, and the protein samples were purified by passing through an equilibrated Q anion-exchange column (ion-exchange pre-packed column HiTrap Q HP, 5 ml, GE healthcare) at a flow rate of 3 mL/min. After passing through the anion-exchange column, the sample in the flowing process was collected.

The collected sample from the Q-column was concentrated and then purified with an equilibrated SP cation exchange column (cation exchange column HiTrap SP HP, 5 mL, GE healthcare) at a flow rate of 3 mL/min. Then the loaded column was washed with buffer 3 which is at pH 6.6 and contains 50 mM KPO4, 50 mM NaCl and 5% glycerol, for 10 CV, followed by linear elution with buffer 4 which is at pH 6.6 and contains 50 mM KPO4, 1 M NaCl and 5% glycerol. The collected protein corresponding to the first elution peak was the target protein.

The collected target protein was the purified KOD DNA polymerase fusion protein.

The purified KOD DNA polymerase fusion protein was subjected to SDS-PAGE (5% stacking gel and 12% separation gel). As be seen, the purified KOD DNA polymerase fusion protein was of a size of 91.5 KDa, which was consistent with the expected size.

As the control group, a blank vector pD441-pelB was introduced into *E. coli* BL21 to obtain BL21/pD441-pelB. With the above expression and purification methods, there was no target protein of about 90.5 KDa obtained in the control group.

### 1.2 Preparation of fusion protein of KOD DNA polymerase mutant

The KOD DNA polymerase mutant proteins were prepared as follows.

### 1.2.1 Preparation of recombinant vector expressing KOD DNA polymerase mutant

Primers for the mutants were designed and synthesized by Liuhe BGI Co.,Ltd. KOD DNA polymerase mutant protein was obtained in accordance with a method of fixed-site mutation with the template vector KOD_WT, which may also be obtained by other existing methods.

The KOD DNA polymerase mutants are shown in Table 1, and specifically as follows:
A KOD DNA polymerase mutant protein Mut_K1 was obtained by mutating leucine (L) to alanine (A) at position 408, and tyrosine (Y) to alanine (A) at position 409, and alanine (A) to leucine (L) at position 485 of the amino acid sequence of KOD _WT (SEQ ID NO: 1).
A KOD DNA polymerase mutant protein Mut_K2 was obtained by mutating leucine (L) to isoleucine (I) at position 408, and tyrosine (Y) to alanine (A) at position 409 of the amino acid sequence of KOD WT (SEQ ID NO: 1).
A KOD DNA polymerase mutant protein Mut_K3 was obtained by mutating leucine (L) to alanine (A) at position 408, tyrosine (Y) to alanine (A) at position 409, and glutamic acid (E) to leucine (L) at position 485 of the amino acid sequence of KOD mutant KOD_GH78 (SEQ ID NO: 5).
A KOD DNA polymerase mutant protein Mut_K4 was obtained by mutating leucine (L) to tyrosine (Y) at position 408 and tyrosine (Y) to alanine (A) at position 409 of the amino acid sequence of KODWT (SEQ ID NO: 1).
A KOD DNA polymerase mutant protein Mut_K5 was obtained by mutating leucine (L) to alanine (A) at position 408, and tyrosine (Y) to alanine (A) at position 409, and glutamic acid (E) to leucine (L) at position 485, and serine (S) to threonine (T) at position 451 of the amino acid sequence of KOD mutant KOD GH78 (SEQ ID NO: 5).
A KOD DNA polymerase mutant protein Mut_K6 was obtained by mutating leucine (L) to alanine (A) at position 408, and tyrosine (Y) to alanine (A) at position 409, and glutamic acid (E) to leucine (L) at position 485, and serine (S) to threonine (L) at position 451 of the amino acid sequence of the KOD mutant KOD _GH78 (SEQ ID NO: 5).
A KOD DNA polymerase mutant protein Mut_K7 was obtained by mutating leucine (L) to alanine (A) at position 408, tyrosine (Y) to alanine (A) at position 409, and proline (P) to aspartic acid (D) at position 410 of the amino acid sequence of KOD _WT (SEQ ID NO: 1).
A KOD DNA polymerase mutant protein Mut_K8 was obtained by mutating leucine (L) to isoleucine (I) at position 408, and tyrosine (Y) to alanine (A) at position 409, and proline (P) to aspartic acid (D) at position 410, and glutamic acid (E) to isoleucine (I) at position 485, and serine (S) to threonine (T) at position 451 of the amino acid sequence of the KOD mutant KOD_GH78 (SEQ ID NO: 5).
A KOD DNA polymerase mutant protein Mut_K9 was obtained by mutating leucine (L) to isoleucine (I) at position 408, and tyrosine (Y) to alanine (A) at position 409, and proline (P) to cysteine (C) at position 410, and glutamic acid (E) to isoleucine (I) at position 485, and serine (S) to leucine (L) at position 451 of the amino acid sequence of the KOD mutant KOD_GH78 (SEQ ID NO: 5).
A KOD DNA polymerase mutant protein Mut_K10 was obtained by mutating leucine (L) to alanine (A) at position 408, tyrosine (Y) to alanine (A) at position 409, proline (P) to cysteine (C) at position 410, and glutamic acid (E) to leucine (L) at position 485 , and serine (S) to threonine (T) at position 451 of the amino acid sequence of the KOD mutant KOD_GH78(SEQ ID NO: 5).
A KOD DNA polymerase mutant protein Mut_K11 was obtained by mutating leucine (L) to alanine (A) at position 408, tyrosine (Y) to alanine (A) at position 409, proline (P) to aspartic acid (D) at position 410, and glutamic acid (E) to leucine (L) at position 485, and serine (S) to threonine (T) at position 451 of the amino acid sequence of the KOD mutant KOD_GH78 (SEQ ID NO: 5).
A KOD DNA polymerase mutant protein Mut_K12 was obtained by mutating leucine (L) to alanine (A) at position 408, and tyrosine (Y) to alanine (A) at position 409, and proline (P) to asparagine (N) at position 410, and glutamic acid (E) to leucine (L) at position 485, and serine (S) to threonine (T) at position 451 of the amino acid sequence of the KOD mutant KOD_GH78 (SEQ ID NO: 5).
A KOD DNA polymerase mutant protein Mut_K13 was obtained by mutating leucine (L) to isoleucine (I) at position 408, and tyrosine (Y) to alanine (A) at position 409, alanine (A) to glutamate (E) at position 485, valine (V) to histidine (H) at position 589, and threonine (T) to lysine (K) at position 676, and valine (V) to methionine (M) at position 680 of the amino acid sequence of the KODWT amino acid sequence (SEQ ID NO: 1).
A KOD DNA polymerase mutant protein Mut_K14 was obtained by mutating leucine (L) to isoleucine (I) at position 408, and tyrosine (Y) to alanine (A) at position 409, and alanine (A) to glutamic acid (E) at position 485, and serine (S) to threonine (T) at position 383, and tyrosine (Y) to phenylalanine (F) at position 384, and valine (V) to isoleucine (I) at position 389 of the amino acid sequence of the KODWT sequence (SEQ ID NO: 1).
A KOD DNA polymerase mutant protein Mut_K15 was obtained by mutating leucine (L) to isoleucine (I) at position 408, tyrosine (Y) to alanine (A) at position 409, alanine (A) to glutamate (E) at position 485, serine (S) to threonine (T) at position 451, and valine (V) to histidine (H) at position 589, threonine (T) to lysine (K) at position 676, and valine (V) to methionine (M) at position 680 of the amino acid sequence of the KODWT sequence (SEQ ID NO: 1).
A KOD DNA polymerase mutant protein MuT_K16 was obtained by mutating leucine (L) to isoleucine (I) at position 408, tyrosine (Y) to alanine (A) at position 409, alanine (A) to glutamate (E) at position 485, serine (S) to leucine (L) at position 451, and Serine (S) to threonine (T) at position 383, tyrosine (Y) to phenylalanine (F)at position 384, and valine (V) to isoleucine (I) at position 389 of the amino acid sequence of the KODWT sequence (SEQ ID NO: 1).
AKOD DNA polymerase mutant protein MuT_K17 was obtained by mutating leucine (L) to alanine (A) at position 408, and tyrosine (Y) to alanine (A) at position 409, and glutamic acid (E) to leucine (L) at position 485, and serine (S) to threonine (T) at position 451, and tyrosine (Y) to phenylalanine (F) at position 493, and tyrosine (Y) to valine (V)at position 497 of the amino acid sequence of the KOD mutant KOD _GH78 (SEQ ID NO: 5).
AKOD DNA polymerase mutant protein MuT_K18 was obtained by mutating leucine (L) to alanine (A) at position 408, and tyrosine (Y) to alanine (A) at position 409, and tyrosine (Y) to leucine (L) at position 497 of the KOD WT sequence (SEQ ID NO: 1).
AKOD DNA polymerase mutant protein MuT_K19 was obtained by mutating leucine (L) to alanine (A) at position 408, and tyrosine (Y) to alanine (A) at position 409, and alanine (A) to leucine (L) at position 485, and serine (S) to threonine (T) at position 451of the KOD WT sequence (SEQ ID NO: 1).
A KOD DNA polymerase mutant protein MuT_K20 was obtained by mutating leucine (L) to alanine (A) at position 408, and tyrosine (Y) to alanine (A) at position 409, and glutamic acid (E) to leucine (L) at position 485, and serine (S) to threonine (T) at position 451, and tyrosine (Y) to phenylalanine (F) at position 493 and tyrosine (Y) to phenylalanine (F) at position 497 of the amino acid sequence of the KOD mutant KOD_GH78 (SEQ ID NO: 5).
AKOD DNA polymerase mutant protein MuT_K21 was obtained by mutating leucine (L) to alanine (A) at position 408, and tyrosine (Y) to alanine (A) at position 409, and glutamic acid (E) to leucine (L) at position 485, and serine (S) to threonine (T) at position 451, and isoleucine (I) to arginine (R) at position 486 of the amino acid sequence of the KOD mutant KOD_GH78 (SEQ ID NO: 5).
A KOD DNA polymerase mutant MuT_K22 was obtained by mutating leucine (L) to alanine (A) at position 408, tyrosine (Y) to alanine (A) at position 409, glutamic acid (E) to leucine (L) at position 485, serine (S) to threonine (T) at position 451, and isoleucine (I) to threonine (T) at position 474, and leucine (L) to isoleucine (I) at position 478 of the amino acid sequence of the KOD mutant KOD _GH78 (SEQ ID NO: 5).
A KOD DNA polymerase mutant protein MuT_K23 was obtained by mutating leucine (L) to alanine (A) at position 408, and tyrosine (Y) to alanine (A) at position 409, and glutamic acid (E) to leucine (L) at position 485, and serine (S) to threonine (T) at position 451, and isoleucine (I) to threonine (T) at position 474, and leucine (L) to phenylalanine (F) at position 478 of the amino acid sequence of the KOD mutant KOD _GH78 (SEQ ID NO: 5).
A KOD DNA polymerase mutant protein MuT_K24 was obtained by mutating leucine (L) to alanine (A) at position 408, and tyrosine (Y) to alanine (A) at position 409, and glutamic acid (E) to leucine (L) at position 485, and serine (S) to threonine (T) at position 451, and aspartic acid (D) to threonine (T) at position 480 of the amino acid sequence of the KOD mutant KOD_GH78 (SEQ ID NO: 5).
A KOD DNA polymerase mutant protein MuT_K25 was obtained by mutating leucine (L) to alanine (A) at position 408, and tyrosine (Y) to alanine (A) at position 409, and glutamate (E) to isoleucine (I) at position 485, and serine (S) to threonine (T) at position 451, and aspartic acid (D) to asparagine (N) at position 480 of the amino acid sequence of the KOD mutant KOD_GH78 (SEQ ID NO: 5).
A KOD DNA polymerase mutant protein MuT_K26 was obtained by mutating leucine (L) to alanine (A) at position 408, and tyrosine (Y) to alanine (A) at position 409, and glutamic acid (E) to isoleucine (I) at position 485, and serine (S) to threonine (T) at position 451, and arginine (R) to cysteine (C) at position 484 of the amino acid sequence of the KOD mutant KOD_GH78 (SEQ ID NO: 5).
A KOD DNA polymerase mutant protein MuT_K27 was obtained by mutating leucine (L) to alanine (A) at position 408, tyrosine (Y) to alanine (A) at position 409, glutamic acid (E) to leucine (L) at position 485, serine (S) to threonine (T) at position 451, and leucine (L) to arginine (R) at position 478 of the amino acid sequence of the KOD mutant KOD_GH78 (SEQ ID NO: 5).
A KOD DNA polymerase protein mutant MuT_K28 was obtained by mutating leucine (L) to alanine (A) at position 408, tyrosine (Y) to alanine (A) at position 409, glutamic acid (E) to leucine (L) at position 485, serine (S) to threonine (T) at position 451, and leucine (L) to phenylalanine (F) at position 478 of the amino acid sequence of the KOD mutant KOD_GH78 (SEQ ID NO: 5).
A KOD DNA polymerase mutant protein MuT_K29 was obtained by mutating leucine (L) to alanine (A) at position 408, and tyrosine (Y) to alanine (A) at position 409, and glutamic acid (E) to leucine (L) at position 485, and serine (S) to threonine (T) at position 451, and isoleucine (I) to lysine (K) at position 474, and leucine (L) to arginine (R) at position 478 of the amino acid sequence of the KOD mutant KOD _GH78 (SEQ ID NO: 5).
AKOD DNA polymerase mutant protein MuT_K30 was obtained by mutating leucine (L) to alanine (A) at position 408, and tyrosine (Y) to alanine (A) at position 409, and glutamic acid (E) to leucine (L) at position 485, serine (S) to threonine (T) at position 451, and isoleucine (I) to lysine (K) at position 474, and leucine (L) to isoleucine (I) at position 478 of the amino acid sequence of the KOD mutant KOD _GH78 (SEQ ID NO: 5).
A KOD DNA polymerase mutant protein MuT_K31 was obtained by mutating leucine (L) to alanine (A) at position 408, and tyrosine (Y) to alanine (A) at position 409, and glutamic acid (E) to leucine (L) at position 485, and serine (S) to threonine (T) at position 451, and isoleucine (I) to lysine (K) at position 474, and leucine (L) to glycine (G) at position 478 of the amino acid sequence of the KOD mutant KOD _GH78 (SEQ ID NO: 5).
AKOD DNA polymerase mutant protein MuT_K32 was obtained by mutating leucine (L) to alanine (A) at position 408, and tyrosine (Y) to alanine (A) at position 409, and glutamic acid (E) to leucine (L) at position 485, and serine (S) to threonine (T) at position 451, and isoleucine (I) to threonine (T) at position 474, and leucine (L) to histidine (H) at position 478 of the amino acid sequence of the KOD mutant KOD _GH78 (SEQ ID NO: 5).
AKOD DNA polymerase mutant protein MuT_K33 was obtained by mutating leucine (L) to alanine (A) at position 408, and tyrosine (Y) to alanine (A) at position 409, and glutamic acid (E) to leucine (L) at position 485, and serine (S) to threonine (T) at position 451, and isoleucine (I) to threonine (T) at position 474, and leucine (L) to cysteine (C) at position 478 of the amino acid sequence of the KOD mutant KOD _GH78 (SEQ ID NO: 5).
A KOD DNA polymerase mutant protein MuT_K34 was obtained by mutating the amino acid leucine (L) to alanine (A) at position 408, and the amino acid tyrosine (Y) to alanine (A) at position 409, and the amino acid glutamic acid (E) to leucine (L) at position 485, and serine (S) to threonine (T) at position 451, and the amino acid isoleucine (I) to cysteine (T) at position 474, and leucine (L) to tyrosine (Y) at position 478 of the amino acid sequence of the KOD mutant KOD_GH78 (SEQ ID NO: 5).
AKOD DNA polymerase mutant protein MuT_K35 was obtained by mutating leucine (L) to alanine (A) at position 408, and tyrosine (Y) to alanine (A) at position 409, and glutamic acid (E) to leucine (L) at position 485, and serine (S) to threonine (T) at position 451, and isoleucine (I) to threonine (T) at position 474, and leucine (L) to serine (S) at position 478 of the amino acid sequence of the KOD mutant KOD _GH78 (SEQ ID NO: 5).

The recombinant vector expressing different KOD DNA polymerase mutant was obtained by recombining a coding gene of different KOD DNA polymerase mutant proteins which is fused with His tags into a vector pD441-pelB. The mutant proteins encoding genes with different point mutations and fused with His tags were expressed through signal peptides in the vector pD441-pelB.

The amino acid sequence of each fusion protein of KOD DNA polymerase mutant is a sequence of the KOD DNA polymerase mutant protein connected with six His tags to the C-terminus thereof.

The nucleotide sequence of the coding gene of different KOD DNA polymerase mutant protein fused with His tags is a sequence of the coding gene of different KOD DNA polymerase mutant connected with six His tag codons at the 3' end thereof.

### 1.2.2 Construction of recombinant bacterium

According to the same methods as described in 1.1.2, the recombinant vectors expressing different KOD DNA polymerase mutants prepared in the 1.2.1 were introduced into BL21 to obtain the recombinant bacteria expressing the fusion proteins of different KOD DNA polymerase mutants respectively.

### 1.2.3 Expression and purification of mutants

According to the same methods as described in 1.1, the recombinant bacteria expressing different fusion proteins of the KOD DNA polymerase mutants prepared in 1.2.2 above were expressed and purified to obtain different fusion proteins of KOD DNA polymerase mutants.

The different fusion proteins of the KOD DNA polymerase mutants were detected by SDS-PAGE with 5% stacking gel and 12% separation gel to obtain target proteins.

The concentrations of KOD DNA polymerase mutant proteins were determined with Bradford Protein Concentration Assay Kit (Thermo Fisher).

### Example 2: Preparation of 9°N DNA polymerase and mutant protein thereof

### 2.1 Preparation of 9°N DNA polymerase

The amino acid sequence of the 9°N DNA polymerase 9°N_WT was set forth as SEQ ID NO: 2 in the sequence listing.

The preparation method was the same as that of 1.1 of Example 1, differing only in that the gene encoding the KOD DNA polymerase and fused with His tags was replaced with a gene encoding the 9°N DNA polymerase and fused with His tags.

The nucleotide sequence of His-tag fused 9°N DNA polymerase coding gene was a sequence of a gene encoding 9°N DNA polymerase 9°N_WT as shown in SEQ ID NO: 2 connected to six His-tag codons at the 3' end thereof.

The purified 9°N DNA polymerase fusion protein was of a size of about 90.5 KDa, which was consistent with the expected size.

### 2.2 Preparation of fusion protein of 9°N DNA polymerase mutant

The preparation method is the same as that of 1.2 in Example 1, differing only in that the different KOD DNA polymerase mutant protein-coding genes fused with His tags were replaced with different 9°N DNA polymerase mutant protein-coding genes fused with His tags.

The different 9°N DNA polymerase mutant proteins are shown in Table 1, and specifically as follows.

A 9°N DNA polymerase protein mutant Mut_N1 was obtained by mutating leucine (L) to alanine (A) at position 408, and tyrosine (Y) to alanine (A) at position 409, and alanine (A) to leucine (L) at position 485 of the amino acid sequence of 9°N_WT (SEQ ID NO: 2).

A 9°N DNA polymerase mutant protein Mut_N2 was obtained by mutating leucine (L) to alanine (A) at position 408, and tyrosine (Y) to alanine (A) at position 409, and serine (S) to leucine (L) at position 451, and alanine (A) to leucine (L) at position 485 of the amino acid sequence of 9°N_WT (SEQ ID NO: 2).

A 9°N DNA polymerase mutant protein Mut_N3 was obtained by mutating leucine (L) to alanine (A) at position 408, and tyrosine (Y) to alanine (A) at position 409, alanine (A) to leucine (L) at position 485, and tyrosine (Y) to leucine (L) at position 497 of the amino acid sequence of 9°N_WT (SEQ ID NO: 2).

A 9°N DNA polymerase mutant protein Mut_N4 was obtained by mutating leucine (L) to alanine (A) at position 408, and tyrosine (Y) to alanine (A) at position 409, and proline (P) to asparagine (N) at position 410, and alanine (A) to leucine (L) at position 485 of the amino acid sequence of 9°N_WT (SEQ ID NO: 2).

A 9°N DNA polymerase mutant protein Mut_N5 was obtained by mutating leucine (L) to alanine (A) at position 408, tyrosine (Y) to alanine (A) at position 409, proline (P) to aspartic acid (D) at position 410, serine (S) to the leucine (L) at position 451, alanine (A) to leucine (L) at position 485 of the amino acid sequence of 9°N_WT (SEQ ID NO: 2).

A 9°N DNA polymerase mutant protein Mut_N6 was obtained by mutating leucine (L) to alanine (A) at position 408, and tyrosine (Y) to alanine (A) at position 409, and alanine (A) to leucine (L) at position 485, and valine (V) to histidine (H) at position 589, and threonine (T) to lysine (K) at position 676, and valine (V) to methionine (M) at position 680 of the amino acid sequence of 9°N_WT (SEQ ID NO: 2).

A 9°N DNA polymerase mutant protein Mut_N7 was obtained by mutating leucine (L) to alanine (A) at position 408, and tyrosine (Y) to alanine (A) at position 409, and alanine (A) to leucine (L) at position 485, and serine (S) to threonine (T) at position 383, and tyrosine (Y) to phenylalanine (F) at position 384, and valine (V) to isoleucine (I) at position 389 of the amino acid sequence of 9°N_WT (SEQ ID NO: 2).

A 9°N DNA polymerase mutant protein Mut_N8 was obtained by mutating leucine (L) to isoleucine (I) at position 408, and tyrosine (Y) to alanine (A) at position 409, and alanine (A) to isoleucine (I) at position 485, and serine (S) to threonine (T ) at position 383, tyrosine (Y) to phenylalanine (F) at position 384, valine (V) to isoleucine (I) at position 389, and valine (V) to histidine (H) at position 589, threonine (T) to lysine (K) at position 676, and valine (V) to methionine (M) at position 680 of the amino acid sequence of 9°N_WT (SEQ ID NO: 2).

A 9°N DNA polymerase mutant protein Mut_N9 was obtained by mutating leucine (L) to isoleucine (I) at position 408, and tyrosine (Y) to alanine (A) at position 409, and the serine (S) to the leucine (L) at position 451, and the alanine (A) to isoleucine (I) at position 485, and serine (S) to threonine (T) at position 383, tyrosine (Y) to phenylalanine (F) at position 384, valine (V) to isoleucine (I) at position 389, and valine (V) to histidine (H) at position 589, threonine (T) to lysine (K) at position 676, and valine (V ) to methionine (M) at position 680 of the amino acid sequence of 9°N_WT (SEQ ID NO: 2).

A 9°N DNA polymerase mutant protein Mut_N10 was obtained by mutating leucine (L) to alanine (A) at position 408, and tyrosine (Y) to alanine (A) at position 409, and alanine (A) to leucine (L) at position 485, and alanine (A) to glycine (G) at position 491, tyrosine (Y) to phenylalanine (F) at position 494, and tyrosine (Y) to leucine (L) at position 497 of the amino acid sequence of 9°N_WT (SEQ ID NO: 2).

### Example 3: Preparation of TGO DNA polymerase TGO_WT and mutant proteins thereof

### 3.1 Preparation of TGO DNA polymerase TGO _WT

The amino acid sequence of the TGO DNA polymerase TGO_WT was set forth as SEQ ID NO: 3 in the sequence listing.

The preparation method was the same as that of 1.1 of Example 1, differing only in that the gene encoding the KOD DNA polymerase and fused with His tags was replaced with a gene encoding TGO DNA polymerase and fused with His tags.

The nucleotide sequence of His-tag fused TGO DNA polymerase coding gene was a sequence of the gene encoding TGO DNA polymerase TGO_WT as shown in SEQ ID NO: 3 connected to six His-tag codons at the 3' end thereof.

The purified TGO DNA polymerase fusion protein was of a size of 90.5 KDa, which was consistent with the expected size.

### 3.2 Preparation of fusion protein of TGO DNA polymerase mutant

The preparation method is the same as that of 1.2 in Example 1, differing only in that the genes encoding different KOD DNA polymerase mutant proteins and fused with His tags were replaced with genes encoding different TGO DNA polymerase mutant proteins and fused with His tags.

The different TGO DNA polymerase mutant proteins are shown in Table 1, and specifically as follows.

A TGO DNA polymerase mutant protein Mut_T1 was obtained by mutating leucine (L) to alanine (A) at position 408, and tyrosine (Y) into alanine (A) at position 409 of the amino acid sequence of TGO_WT (SEQ ID NO: 3).

A TGO DNA polymerase mutant protein Mut_T2 was obtained by mutating leucine (L) to isoleucine (I) at position 408, and tyrosine (Y) into alanine (A) at position 409 of the amino acid sequence of TGO_WT (SEQ ID NO: 3).

A TGO DNA polymerase mutant protein Mut_T3 was obtained by mutating leucine (L) to isoleucine (I) at position 408, and tyrosine (Y) into alanine (A) at position 409, alanine (A) into isoleucine (I) at position 485, and serine (S) to leucine (L) at position 451 of the amino acid sequence of TGO_WT (SEQ ID NO: 3).

A TGO DNA polymerase mutant protein Mut_T4 was obtained by mutating leucine (L) to isoleucine (I) at position 408, and tyrosine (Y) into alanine (A) at position 409, and proline (P) into glycine (G) at position 491 of the amino acid sequence of TGO_WT (SEQ ID NO: 3).

A TGO DNA polymerase mutant protein Mut_T5 was obtained by mutating leucine (L) to isoleucine (I) at position 408, and tyrosine (Y) into alanine (A) at position 409, and alanine (A) into leucine (L) at position 485 of the amino acid sequence of TGO_WT (SEQ ID NO: 3).

A TGO DNA polymerase mutant protein Mut_T6 was obtained by mutating leucine (L) to alanine (A) at position 408, and tyrosine (Y) into alanine (A) at position 409, proline (P) in asparagine (N) at position 410 of the amino acid sequence of TGO_WT (SEQ ID NO: 3).
A TGO DNA polymerase mutant protein Mut_T7 of was obtained by mutating leucine (L) to alanine (A) at position 408, and tyrosine (Y) into alanine (A) at position 409, and valine (V) into glutamine (Q) at position 93, and alanine (A) to leucine (L) at position 485 of the amino acid sequence of TGO_WT (SEQ ID NO: 3).

### Example 4: Preparation of TOK DNA polymerase TOK_WT and mutant proteins thereof

### 4.1 Preparation of TOK DNA polymerase

The amino acid sequence of TOK_WT was set forth as SEQ ID NO: 4 in the sequence listing.

The preparation method was the same as that of 1.1 of Example 1, differing only in that the gene encoding the KOD DNA polymerase and fused with His tags was replaced with a gene encoding the TOK DNA polymerase and fused with His tags.

The nucleotide sequence of the Hig-tag fused TOK DNA polymerase coding gene was a sequence of the gene encoding TOK DNA polymerase TOK _WT as shown in SEQ ID NO: 4 connected to six His-tag codons at the 3' end thereof.

The purified TOK DNA polymerase fusion protein was of a size of about 90.7 KDa, which was consistent with the expected size.

### 4.2 Preparation of fusion protein of TOK DNA polymerase mutant

The preparation method is the same as that of 1.2 in Example 1, differing only in that the genes encoding different KOD DNA polymerase mutant proteins and fused with His tags were replaced with genes encoding different TOK DNA polymerase mutant proteins and fused with His tags.

The different TOK DNA polymerase mutant proteins are shown in Table 1, and specifically as follows.

A TOK DNA polymerase mutant protein Mut_O1 was obtained by mutating leucine (L) to alanine (A) at position 408, and tyrosine (Y) to alanine (A) at position 409 of the amino acid sequence of TOK_WT (SEQ ID NO: 4).

A TOK DNA polymerase mutant protein Mut_O2 was obtained by mutating leucine (L) to isoleucine (I) at position 408, and tyrosine (Y) to alanine (A) at position 409 of the amino acid sequence of TOK_WT (SEQ ID NO: 4).

A TOK DNA polymerase mutant protein Mut_O3 was obtained by mutating leucine (L) to isoleucine (I) at position 408, and tyrosine (Y) to alanine (A) at position 409, and alanine (A) to isoleucine (I) at position 485, and serine (S) to leucine (L) at position 451 of the amino acid sequence of TOK_WT (SEQ ID NO: 4).

### Example 5: Detection of the performance of DNA polymerases including KOD_WT, 9°N _WT, TGO_WT and their mutants

### 5.1 Polymerization activity assay of DNA polymerases on incorporating single nucleotide with modification

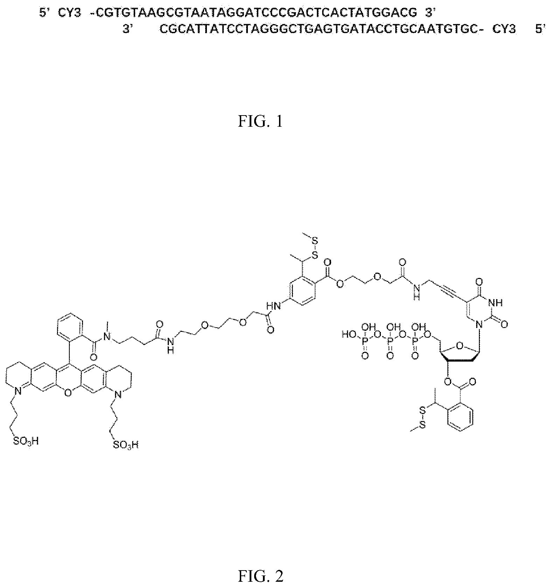

The dTTP with 3'-end blocking modification and Cy5 fluorescent dye labeled as shown in FIG. 2 and the DNA strand labeled with Cy3 fluorescent dye (DNA-Cy3, BGI) were used in this Example, where the sequence of DNA-Cy3 may refer to FIG. 1.

Incorporations of the modified nucleotides in high-throughput sequencing were simulated. During the process, a relative reaction rate of the polymerase mutant was detected with a microplate reader to calculate an activity of the crude enzyme.

The specific protocol was as follows.

The reaction buffer contained 20 mM Tris-HCl, 10 mM (NH4)2SO4, 10 mM KCl, 2 mM MgSO4, with pH 8.5 at 25 °C.

The reaction system was of 1 µg of respective polymerase proteins obtained in Examples 1-4, mixed with a reaction solution containing 2 µM modified dTTP and 1 µM DNA-Cy3.

The reaction solution was formulated as: 20 mM Tris-HCl, 10 mM (NH4)2SO4, 10 mM KCl, 2 mM MgSO4, 2 µM of dTTP with 3'-end blocking modification, 1 µM of DNA-Cy3, and water for making up the reaction solution, with pH 8.5.

The reaction was performed at 40 °C for 1 h.

After that, data sheet and enzyme activity curve were exported directly to calculate the activity of the crude enzyme.

KOD mutant GH78 (SEQ ID NO: 5) was used as a reference, i.e., the relative activity of GH78 was set at 1. All other polymerase mutants were compared to this reference. The experimental results were shown in Table 2 below.

**Table 2 Polymerization activity assay of DNA polymerase mutants on incorporating single nucleotide with modification**

| Mutant ID | Activity relative to GH78 |
|---|---|
| KOD_WT | No signal |
| TOK_WT | No signal |
| TGO_WT | No signal |
| 9°N_WT | No signal |
| GH78 | 1.0 |
| Mut_K7 | 0.8 |
| Mut_K8 | 0.8 |
| Mut_K9 | 1.5 |
| Mut_K4 | 0.2 |
| Mut_K2 | 0.4 |
| Mut_K19 | 9.6 |
| Mut_K1 | 12.6 |
| Mut_K3 | 2.8 |
| Mut_K5 | 4.7 |
| Mut_K6 | 9.1 |
| Mut_K10 | 6.6 |
| Mut_K11 | 3.1 |
| Mut_K12 | 5.4 |
| Mut_K13 | 0.9 |
| Mut_K14 | 20 |
| Mut_K15 | 1.7 |
| Mut_K16 | 2.6 |
| Mut_K17 | 9.2 |
| Mut_K20 | 7.8 |
| Mut_K18 | 2.3 |
| Mut_K21 | 6.9 |
| Mut_K22 | 79 |
| Mut_K23 | 6.1 |
| Mut_K24 | 2.9 |
| Mut_K25 | 0.7 |
| Mut_K26 | 1.9 |
| Mut_K27 | 4.0 |
| Mut_K28 | 1.0 |
| Mut_K29 | 3.2 |
| Mut_K30 | 6.8 |
| Mut_K31 | 5.1 |
| Mut_K32 | 2.9 |
| Mut_K33 | 3.4 |
| Mut_K34 | 2.7 |
| Mut_K35 | 5.4 |
| Mut_N1 | 1.9 |
| Mut_N2 | 0.5 |
| Mut_N3 | 3.9 |
| Mut_N4 | 0.7 |
| Mut_N5 | 0.2 |
| Mut_N6 | 1.1 |
| Mut_N7 | 1.1 |
| Mut_N8 | 2.0 |
| Mut_N9 | 1.2 |
| Mut_N10 | 1.0 |
| Mut_11 | 7.4 |
| Mut_T2 | 0.3 |
| Mut_T3 | 1.3 |
| Mut_T4 | 0.6 |
| Mut_T5 | 0.5 |
| Mut_T6 | 4.9 |
| Mut_T7 | 5.8 |
| Mut_O1 | 0.8 |
| Mut_O2 | 0.2 |
| Mut_O3 | 1.7 |

It could be seen that none of the wild-type DNA polymerases (KOD WT, 9°N_WT, TGO_WT and TOK_WT) could incorporate 3'-end blocking modified dTTP into the DNA template, while such an incorporation could be achieved by the mutant proteins, producing fluorescent signal values. The experimental results showed that the mutant proteins have different polymerization activities for the dNTP with 3'end blocking modification, indicating effective mutations and combinations thereof at different positions in Examples of the present disclosure, and these mutants are of great potential for use in high-throughput sequencing.

### Example 6

The preferable mutants were subjected to sequencing tests on the MGISEQ-500 sequencer (MGI), and the sequencing results were analyzed as follows.

A sequencing library was *Ecoli.fa* library (Part No. 1000005038, MGI Tech co., Ltd).

The sequencing condition was of polymerization for 90 seconds and excision for 60 seconds.

With the same *Ecoli.fa* sequencing library and sequencing condition, 12 mutants were selected for sequencing tests on MGISEQ-500 sequencer for 50 cycles, and the results were shown in Table 3 below.

**Table 3 The sequencing results using DNA polymerase mutants on MGISEQ-500**

| MGISEQ-500 | Reference | CycleNumber | Q30% | ESR% |
|---|---|---|---|---|
| GH78 | *Ecoli.fa* | 50 | 60.16 | 2.8 |
| Mut_N4 | *Ecoli.fa* | 50 | 67.6 | 79.38 |
| Mut_N1 | *Ecoli.fa* | 50 | 62.26 | 65.95 |
| Mut_K6 | *Ecoli.fa* | 50 | 70.72 | 77.29 |
| Mut_K10 | *Ecoli.fa* | 50 | 60.3 | 71.2 |
| Mut_K3 | *Ecoli.fa* | 50 | 83.59 | 60.84 |
| Mut_K5 | *Ecoli.fa* | 50 | 79.21 | 82.54 |
| Mut_N13 | *Ecoli.fa* | 50 | 71.45 | 79.7 |
| Mut_T7 | *Ecoli.fa* | 50 | 81.23 | 84.27 |
| Mut_N4 | *Ecoli.fa* | 50 | 65.57 | 74.73 |
| Mut_K17 | *Ecoli.fa* | 50 | 77.43 | 80.43 |
| Mut_K20 | *Ecoli.fa* | 50 | 78.83 | 79.93 |
| Mut_K1 | *Ecoli.fa* | 50 | 79.98 | 79.05 |
| Mut_K21 | *Ecoli.fa* | 50 | 75.49 | 79.25 |
| Mut_K19 | *Ecoli.fa* | 50 | 72.64 | 65.11 |
| Mut_K22 | *Ecoli.fa* | 50 | 75.99 | 80.32 |
| Mut_K23 | *Ecoli.fa* | 50 | 76.15 | 85.31 |
| Mut_K31 | *Ecoli.fa* | 51 | 60.69 | 65.73 |

The higher the Q30 and ESR, the better the polymerization effect. According to the sequencing results on MGISEQ-500, the wild-type DNA polymerases (KOD_WT, 9°N_WT, TGO_WT and TOK_WT) present no signal, while the designed mutants had good polymerization activity for the new dNTP with 3'-end blocking modification, where the ESR and Q30 were qualitatively improved compared with GH78, especially Mut_K5, Mut_T7, Mut_K1 and Mut_K20 showed substantial improvement for the ESR and Q30. The results of sequencing with these mutants showed that these mutants were in great potential in use for high-throughput sequencing, indicating the feasibility and innovation of the technical concept of the mutants provided in Examples of the present disclosure, as well as providing an experimental basis for future applications.

### Industrial application

Experiments of the present disclosure showed that the present disclosure analyzed and designed mutants in terms of positions in group A (408/409/410), group B (451/485), group C (389/383/384), group D (589/676/680), group E (491/493/494/497), and group F (474/478/486/480/484) of several B family DNA polymerase. Through site-specific mutagenesis, induced expression and purification, the mutant proteins were subjected to polymerization activity test with the nucleotide having 3'-end blocking modification on microtemplate reader, and then the sequencing test, thereby developing DNA polymerases suitable for sequencing DNA nanoball (DNB) attached on a surface of a chip. Compared with non-mutated DNA polymerase, mutated DNA polymerase in the several groups exhibited strong polymerization activity in catalyzing incorporation of nucleotides with 3'-end blocking modification, providing a foundation for sustainable development of high-throughput sequencing.

## Claims

1. A mutant protein, based on a thermostable B-family DNA polymerase and having DNA polymerase activity, wherein the mutant protein is obtained by mutating at least two amino acid residues in an amino acid sequence of the thermostable B-family DNA polymerase at positions selected from the following three positions in group A: positions 408, 409, and 410.

2. The mutant protein according to claim 1, wherein the mutant protein having DNA polymerase activity, based on the mutant protein of claim 1, is further mutated at least one amino acid residue at a position(s) selected from positions 451 and 485 in group B in the amino acid sequence of the thermostable B-family DNA polymerase.

3. The mutant protein according to claim 2, wherein the mutant protein having DNA polymerase activity, based on the mutant of claim 2, is further mutated at least one amino acid residue at a position(s) selected from three positions 383, 384, and 389 in group C in the amino acid sequence of the thermostable B-family DNA polymerase.

4. The mutant protein according to claim 2 or 3, wherein the mutant protein having DNA polymerase activity, based on the mutant of claim 2 or 3, is further mutated at least one amino acid residue at a position(s) selected from three positions 589, 676, and 680 in group D in the amino acid sequence of the thermostable B-family DNA polymerase.

5. The mutant protein according to any one of claims 2 to 4, wherein the mutant protein having DNA polymerase activity, based on the mutant of claim 2, is further mutated at least one amino acid residue at a position(s) selected from four positions 491, 493, 494, and 497 in group E in the amino acid sequence of the thermostable B-family DNA polymerase.

6. The mutant protein according to claim 2, wherein the mutant protein having DNA polymerase activity, based on the mutant of claim 2, is further mutated at least one amino acid residue at a position(s) selected from five positions 474, 478, 480, 484 and 486 in the F group in the amino acid sequence of the thermostable B-family DNA polymerase.

7. The mutant protein according to claim 2, wherein the mutant protein having DNA polymerase activity, based on the mutant of claim 2, is further mutated an amino acid residue at position 93 in the amino acid sequence of the thermostable B-family DNA polymerase.

8. The mutant protein according to any one of claims 1 to 7, wherein the amino acid at each of the positions is mutated as follows:
L at position 408 is mutated into A, I or Y;
Y at position 409 is mutated into A;
P at position 410 is mutated into D, C or N;
S at position 451 is mutated into T or L;
E at position 485 is mutated into I or L, or A at position 485 is mutated into L or E or I;
I at position 486 is mutated into R;
V at position 93 is mutated into Q;
D at position 480 is mutated into T or N;
R at position 484 is mutated into C;
I at position 474 is mutated into T or K;
L at position 478 is mutated into I or F or R or G or H or C or Y or S;
I at position 474 is mutated into T;
L at position 478 is mutated into I or F;
S at position 383 is mutated into T;
Y at position 384 is mutated to F;
V at position 389 is mutated to I;
V at position 589 is mutated into H;
T at position 676 is mutated into K;
V at position 680 is mutated to M;
A or P at position 491 is mutated into G;
Y at position 493 is mutated to F;
Y at position 494 is mutated to F;
Y at position 497 is mutated into V or F or L.

9. The mutant protein according to claim 1, wherein the thermostable B-family DNA polymerase is a mutant of KOD DNA polymerase, 9°N DNA polymerase, TGO DNA polymerase or TOK DNA polymerase.

10. The mutant protein according to claim 9, wherein the KOD DNA polymerase is of an amino acid sequence set forth in SEQ ID NO: 1, the 9°N DNA polymerase is of an amino acid sequence set forth in SEQ ID NO: 2, the TGO DNA polymerase is of an amino acid sequence set forth in SEQ ID NO: 3, and the TOK DNA polymerase is of an amino acid sequence set forth in SEQ ID NO: 4.

11. The mutant protein according to any one of claims 1 to 10, wherein the mutant protein is added with a tag sequence at an end of an amino acid sequence of the mutant protein according to any one of claims 1 to 8, and the mutant protein has thermostable B-family DNA polymerase activity.

12. A DNA molecule encoding a mutant protein of any one of claims 1 to 10, or
an expression cassette, a recombinant vector, a recombinant bacterium or a transgenic cell line comprising the DNA molecule.

13. Use of a mutant protein of any one of claims 1 to 10 in at least one of the following i to v:
i. performing as a DNA polymerase;
ii. catalyzing DNA replication and/or DNA amplification;
iii. nucleic acid sequencing, wherein a specific dNTP with 3'-end blocking modification is introduced during the sequencing;
iv. preparing a product for catalyzing DNA replication and/or DNA amplification;
v. preparing a product for nucleic acid sequencing, wherein the specific dNTP with 3'-end blocking modification is introduced during the sequencing; or
use of a DNA molecule, an expression cassette, a recombinant vector, a recombinant bacterium or a transgenic cell line of claim 12 in at least one of the following vi to xi:
vi. preparing as a DNA polymerase;
vii. catalyzing DNA replication and/or DNA amplification;
viii. nucleic acid sequencing, wherein a specific dNTP with 3'-end blocking modification is introduced during the sequencing;
ix. preparing a product comprising DNA polymerase;
x. preparing a product for catalyzing DNA replication and/or DNA amplification;
xi. preparing a product for nucleic acid sequencing, wherein the specific dNTP with 3'-end blocking modification is introduced during the sequencing.
